# EUROPEAN PATENT APPLICATION

(11) **EP 4 728 871 A1**
(43) Date of publication of application: **22.04.2026**
(21) Application number: 24306719.6
(22) Date of filing: 17.10.2024
(51) Int. Cl.: A23C 9/123, A23C 11/10, A23L 11/50, C12N 1/20

(54) **STREPTOCOCCUS THERMOPHILUS STRAIN FOR USE IN PREPARATION OF FERMENTED PRODUCTS**

(71) Applicant: Compagnie Gervais Danone, 75009 Paris (FR)
(72) Inventor: SURIYA, Pradeep, Louisville, CO 80027 (US); BAE, Sang, Louisville, CO 80027 (US); COMASIO, Andrea, 91190 GIF-SUR-YVETTE (FR); MARCHAL, Laurent, 91190 GIF-SUR-YVETTE (FR)
(74) Representative: Gevers & Orès

(57) **Abstract**

The present invention relates to a novel strain of *Streptococcus thermophilus,* compositions comprising said strains and to methods for the preparation of such compositions.

## Description

### Field of the invention

The present invention relates to a novel strain of *Streptococcus thermophilus,* compositions comprising said strain and methods for the preparation of such compositions.

### Technical background

*Streptococcus thermophilus* (also referred to as *Streptococcus salivarius* subsp. *thermophilus* and hereinafter also referred to as *S*. *thermophilus*) is a gram-positive lactic acid bacterium that is widely used in the preparation of fermented milk products. In particular, S. *thermophilus* is commonly used together with *Lactobacillus delbrueckii* subsp. *bulgaricus* (also referred to as *L. bulgaricus*) as a starter culture to prepare yogurts and other types of fermented milk products.

In the fermentation process, *S*. *thermophilus* species not only produces lactic acid, decreases pH and causes milk proteins to coagulate but also strongly affects the quality of fermented milk products by the production of metabolites, which greatly differ from one strain to another. For example, in the context of the manufacture of yogurts, the exopolysaccharides produced by *S*. *thermophilus* play a role of thickener and give the end product a creamy texture appreciated by consumers. WO 01/79500 discloses some genetic operons associated to texturizing properties of *S*. *thermophilus* strains.

Fermented milk products, and in particular yogurts, are widely consumed and appreciated in that they contribute to a healthy diet by providing essential nutrients. However, flavored and sweetened fermented milk products are more popular because unflavored fermented milk products have characteristic tart or acidic flavors. In this context, increasing consumer awareness of the benefits of a low-sugar diet together with a trend towards what the consumer perceives as "natural" products has led to a need for the development of fermented milk products with a reduced sugar content. The use of high sugar producing strains of S. *thermophilus* and *L. Bulgaricus* strains has been proposed as a means of increasing the sweetness of fermented milk products. For example, WO 2013/160413 discloses some mutant *S. thermophilus* strains that have sweetening properties due to their secretion of glucose. These strains are characterized by a mutation in the glucokinase (*glcK*) gene and are described as being suitable for use in the preparation of fermented milk products. However, the use of such strains delays the fermentation process by 2-5 hours, requiring at least 20 hours of fermentation. Sorensen et al. (Appl. Environ. Microbiol., 82(12): 3683-3692, 2016) also discloses some mutant *S*. *thermophilus* strains which secrete glucose. However, these mutant strains are not able to acidify milk or display a lag of 2-3 hours over the parent strains in onset of acidification.

### Summary of the invention

The present invention follows from the unexpected finding that a novel strain of *Streptococcus thermophilus* can address texture problems and impact sweetness perception of fermented products. Additionally, this strain is exceptionally useful in the preparation of fermented food products due to its acidification properties. Accordingly, the present invention provides a *Streptococcus thermophilus* strain deposited at the CNCM under reference number CNCM I-2964. The present invention also provides compositions comprising the S. *thermophilus* strain CNCM 1-2964 and methods for the preparation thereof.

### Detailed description of the invention

As used herein the term "stable composition" shall be taken to mean a composition that does not present sedimentation and/or serum separation.

As used herein, the term "ppm" shall be taken to mean "parts per million" One gram in 1 liter is 1000 ppm and one thousandth of a gram (0.001g) in 1 liter is one ppm.

As used herein the term "x% (w/w)" or "x% w/w" is equivalent to "x g per 100 g". Unless indicated otherwise, all % values shall be taken to indicate x% w/w.

As used herein, the term "x% (v/v)" or "x% v/v" is equivalent to "x ml per 100 ml".

In the context of the present invention, the term "at least" also includes the starting point of the open range. For example, an amount of "at least 95.00 % w/w" means any amount equal to 95.00 percentage by weight or above.

In the context of the present invention, the term "about" defines a range of plus or minus 10% of the cited value. For example, an amount of "about 20 weight %" means any amount within the range of 18.00 to 22.00 weight %.

As used herein, the term "vegetal" shall be taken to mean any edible part of a plant, including but not limited to leaves, flowers, stems, fruits, roots and/or tubers.

As used herein, the term "plant-based" shall be taken to mean a composition or product which comprises plant or plant-derived matter but does not comprise animal or animal-derived matter including but not limited to dairy product, egg, fish, shellfish, meat, dairy milk and insects.

As used herein, the adjective "dairy" shall be taken to mean a composition or product which comprises or consists of mammalian milk matter, i.e. the lacteal secretion obtainable by milking.

As used herein the terms "dairy composition", "milk-based composition" or "dairy product" shall be taken to mean a product or composition comprising essentially of or consisting of milk or milk components and optionally further ingredients.

As used herein, the terms "-free" or "free from" shall be taken to mean a composition or product which preferably does not contain a given substance but where trace amounts or contaminants thereof may be present.

As used herein, the term "added sugar" shall refer to sugars that are added during the production or processing of foods (e.g. plant matter processed to provide a vegetal base) as opposed to sugars naturally occurring in said foods. Added sugars include sugars (free, mono- and disaccharides), sugars from syrups and honey, and sugars from concentrated fruit or vegetable juices that are in excess of what would be expected from the same volume of 100 percent fruit or vegetable juice of the same type.

As used herein, the term "fermented plant-based" shall be taken to mean a product or composition that is the product of the acidifying fermentation of a plant-based composition by a starter culture of fermenting microorganisms, in particular bacteria, preferably lactic acid bacteria.

As used herein the term "fermented dairy" shall be taken to mean a product or composition that is the product of the acidifying fermentation of a milk-based composition by a starter culture of fermenting microorganisms, in particular bacteria, preferably lactic acid bacteria. As used herein the term "fermented milk" shall be taken to mean a product or composition derived from milk by the acidifying action of at least one lactic acid bacterium. Accordingly, as used herein a fermented dairy product can thus be a fermented milk, such as a yoghurt (e.g. a set, stirred or drink yogurt), or a fresh cheese such as a white cheese or a *"petit-Suisse".* It can also be a strained fermented milk such as a strained yoghurt (e.g. a concentrated or Greek-style yoghurt).

As used herein, the terms "plant-based" alternative, analogue or substitute shall be taken to mean a plant-based food or beverage composition that is formulated to simulate the organoleptic and/or nutritional qualities of a non plant-based product. Accordingly, a "plant-based fermented milk alternative" shall be taken to mean a plant-based food or beverage composition that is formulated to simulate the organoleptic and/or nutritional qualities of fermented dairy milk. A "plant-based yogurt" shall be taken to mean a plant-based food or beverage composition that is formulated to simulate the organoleptic and/or nutritional qualities of fermented dairy yogurt.

The terms "fermented milk" and "yogurt" or "yoghurt" are given their usual meanings in the field of the dairy industry, that is, products suitable for human consumption and originating from acidifying lactic fermentation of a milk substrate. These products can contain secondary ingredients such as fruits, vegetables, sugar, etc. The expression "fermented milk" may be used to refer to fermented milks other than yogurts e.g. "Kefir", "Kumtss", "Lassi", "Dahi", "Leben", "Filmjolk", "Villi", "Acidophilus milk".

The term "yogurt" or "yoghurt" as used herein shall be taken to mean fermented milk obtained by the acidifying lactic fermentation of specific thermophilic lactic acid bacteria such as *Lactobacillus bulgaricus* and *Streptococcus thermophilus,* which must be viable in the finished product at a minimum CFU. In certain countries, regulations allow the addition of further lactic acid bacteria to yoghurt such as but not limited to strains of *Bifidobacterium* and/or *Lactobacillus acidophilus* and /or *Lactobacillus casei.* These additional lactic acid bacteria strains are intended to impart various properties to the finished product, such as that of providing organoleptic qualities, favoring equilibrium of intestinal flora or modulating the immune system.

As used herein the term "strained fermented food product" shall be taken to mean a food product which has been subjected to a post-fermentation separation process.

As used herein the term "spoonable" shall be taken to mean a solid or semi-solid that may be consumed by means of a spoon or other utensil.

As used herein the term "fermentation" shall be taken to mean the metabolism of a substance by microorganisms, e.g. bacteria, yeasts, or other microorganisms.

As used herein the term "viable" when used in reference to a micro-organism shall be taken to mean a metabolically and physiologically active micro-organism. Means for the detection of viability in micro-organisms are known in the art and include plate count and flow cytometry based methods.

As used herein the term "cfu" or "CFU" shall be taken to be an abbreviation of the term "colony forming unit".

As used herein the term "CNCM I-" followed by a 4 digit number shall be taken to refer to a strain deposited at the Collection Nationale de Cultures de Microorganismes (CNCM), Institut Pasteur, 25 rue du Docteur Roux, 75724 Paris Cedex 15, France, under the Budapest Treaty with an accession number corresponding to said 4 digit number.

As used herein reference to a bacterial strain or species shall be taken to include functionally equivalent bacteria derived therefrom such as but not limited to mutants, variants or genetically transformed bacteria. These mutants or genetically transformed strains can be strains wherein one or more endogenous gene(s) of the parent strain has (have) been mutated, for instance to modify some of their metabolic properties (e.g., their ability to ferment sugars, their resistance to acidity, their survival to transport in the gastrointestinal tract, their post-acidification properties or their metabolite production). They can also be strains resulting from the genetic transformation of the parent strain to add one or more gene(s) of interest, for instance in order to give to said genetically transformed strains additional physiological features, or to allow them to express proteins of therapeutic or prophylactic interest that one wishes to administer through said strains. These mutants or genetically transformed strains can be obtained from the parent strain by means of conventional techniques for random or site-directed mutagenesis and genetic transformation of bacteria, or by means of the technique known as "genome shuffling". In the present text, strains, mutants and variants derived from a parent species or strain will be considered as being encompassed by reference to said parent species or strain, e.g. the phrases *"S*. *thermophilus"* and *"Streptococcus thermophilus* strain CNCM I-2964" shall be taken to include strains, mutants and variants derived therefrom. Accordingly, as used herein reference to a bacterial strain specified by an accession or deposit number shall be taken to encompass variants thereof having at least 95 % identity (see: Stackebrandt & Goebel, 1994, Int. J. Syst. Bacteriol. 44:846-849). In a particularly preferred embodiment, said variant has at least 97 % identity with the 16S rRNA sequence of said specified strain, more preferably at least 98 % identity, more preferably at least 99 % or more identity.

As used herein the term "substantially pure" when used in reference to a bacterial strain refers to the percent of said bacterial strain relative to the total micro-organism content. Substantially pure can be at least about 99.99%, at least about 99.90%, at least about 99.50%, at least about 99.00%, at least about 95.00%, at least about 90.00%, at least about 85.00%, or at least about 75.00%.

As used herein, a "lactic acid bacterium" is a Gram-positive, acid-tolerant, generally non-sporulating and non-respiring, either rod- or cocci-shaped bacterium that is able to ferment sugars into lactic acid.

The present invention relates to a novel strain of *Streptococcus thermophilus,* compositions comprising said strain and to methods for the preparation of such compositions.

### Streptococcus thermophilus strain

In a first aspect the present invention provides a strain of *Streptococcus thermophilus.* In a first embodiment the present invention provides the *Streptococcus thermophilus* strain CNCM I-2964. This strain has been deposited at the Collection Nationale de Cultures de Microorganismes (CNCM) (Institut Pasteur, 25-28 Rue du Docteur Roux, 75724 Paris Cedex 15, France) under the Budapest Treaty on December 19^{th}, 2002 under reference number CNCM I-2964. The deposit was made in accordance with the Budapest Treaty on the International Recognition of the Deposit of Microorganisms for the Purposes of Patent Procedure, as provided therein the applicant requests that a sample of the deposited micro-organisms only be made available to an independent expert, until the date on which the patent may be granted. In one embodiment the present invention provides the isolated strain CNCM I-2964, preferably said isolate is substantially pure.

### Compositions of the invention

In a second aspect the present invention provides compositions comprising the *Streptococcus thermophilus* strain CNCM I-2964. Preferably, the composition comprises at least 10⁶, more preferably at least 10⁷ and most preferably at least 10⁸ colony forming unit (CFU) of *S*. *thermophilus* strain CNCM I-2964 per gram (g) of composition according to embodiments of the invention.

In embodiments, the composition comprises 10⁵ to 10¹² colony forming unit (CFU) of the *Streptococcus thermophilus* strain CNCM I-2964 per gram (g) of composition according to embodiments of the invention. In further embodiments, the composition comprises 10⁶ to 10¹¹ colony forming unit (CFU) of the *Streptococcus thermophilus* strain CNCM I-2964 per gram (g) of composition according to embodiments of the invention.

Advantageously, the composition is a manufactured composition. In addition to the strain *Streptococcus thermophilus* CNCM I-2964, the composition may comprise a medium. The medium may be a milk base fermented or not fermented, a vegetal base fermented or not fermented or any other medium for example a culture medium.

The bacterium as provided herein is suitable for use in edible compositions, accordingly in one embodiment the present invention provides a composition suitable for human consumption or ingestion, preferably by oral means. Accordingly, the composition comprises or consists of comestible matter. It is particularly preferred that the compositions of embodiments of the invention are substantially free of pathogenic or toxicogenic matter. The composition according to embodiments of the invention may be a medicament or pharmaceutical composition. In a particularly preferred embodiment the composition according to the invention may be a non-therapeutic composition, preferably a nutraceutical composition, a nutritional composition and/or a food composition. It is particularly preferred that the food composition is a food product, preferably fermented food product, preferably a fermented plant-based or dairy food product. Further compositions according to embodiments of the invention also include food additives, food ingredients, nutritional formulas, baby foods, infant milk formulas and infant follow-on formulas.

The composition may comprise further additional strains of *Bifidobacterium* and/or lactic acid bacteria; typically 1, 2, 3, 4 or more additional strains. Examples of *Bifidobacterium* that can be used include but are not limited to *Bifidobacterium animalis* (for example *Bifidobacterium animalis* subsp. *animalis* or *Bifidobacterium animalis* subsp. *lactis*); *Bifidobacterium longum*; *Bifidobacterium breve*; *Bifidobacterium bifidum.* Examples of lactic acid bacteria that can be used include but are not limited to *Lactobacilli* (for example *Lactobacillus acidophilus, Lactobacillus buchneri, Lactobacillus delbruckei,* in particular *L. delbrueckii* subsp. *bulgaricus* or *lactis, Lactobacillus casei, Lactobacillus plantarum, Lactobacillus reuteri, Lactobacillus johnsonii, Lactobacillus helveticus, Lactobacillus brevis, Lactobacillus rhamnosus*); *Lactococci* (for example *Lactococcus lactis,* typically *Lactococcus lactis* subsp. *lactis* or *Lactococcus lactis* subsp. *cremoris*)*.* Preferably the composition further comprises *Lactobacillus* and/or *Streptococcus.* For the preparation of yogurt, the composition typically comprises *Lactobacillus bulgaricus* and *Streptococcus thermophilus,* optionally with additional microorganisms such as but not limited to probiotic species or other species that may provide desirable organoleptic or other qualities to the composition, e.g. further strains of *Lactococcus lactis.*

In an embodiment, the composition of the invention does not comprise any strain of *Streptococcus thermophilus* other than the strain CNCM I-2964.

### Fermented food products

The strains of the present invention are particularly suited to the preparation of fermented food products, in embodiments a fermented plant-based and/or dairy food product.

In embodiments the fermented food products of the invention comprise a) fermented milk and/or vegetal base and b) the strain CNCM I-2964.

### a) Vegetal base

In one embodiment, the vegetal base is an aqueous suspension comprising water and plant-matter selected from the group consisting of legumes, nuts, seeds, cereals and/or combination thereof.

In embodiments, the legumes, are pulses. In other embodiments, the pulses are selected from the group consisting of split peas, field peas, dry peas, lentil, chickpeas, garbanzo bean, konda, navy bean, white navy bean, white pea bean, pea bean, cow pea, horse bean, haricot, pinot bean, mottled bean, small red bean, red Mexican bean, kidney bean, black bean, black turtle bean, cranberry bean, roman bean, speckled sugar bean, lima bean, faba bean, Madagascar bean, green gram, mung bean, green bean, black gram, urad dal, soy and/or lupin. In preferred embodiments, the pulses are pea, faba and/or chickpea.

In other embodiments, the nuts are selected from the group consisting of almonds, cashews, pecans, macadamias, hazelnuts, pistachio, walnuts and/or combinations thereof.

In other embodiments, the seeds are selected from the group consisting of hemp, pumpkin, quinoa, sesame, tiger nut, flax, chia, sunflower, coconut and/or combinations thereof.

In other embodiments, said cereals are selected from the group consisting of wheat, rye, spelt, barley, oat, millet, sorghum, rice, teff and/or combinations thereof.

Processes for the preparation of such suspensions are known in the art and typically comprise mechanical and/or enzymatic disruption of the plant-matter and hydration and/or combination with a solution, followed by mechanical separation of an aqueous fraction from starchy and/or fibrous matter, e.g., by decantering, centrifugation or filtration.

For example, the plant-matter may be milled, ground, soaked, dehulled, mixed with water, optionally enzymatic hydrolysed and/or homogenized etc. in order to produce a suitable aqueous composition.

In other embodiments, the plant matter may be a seed or nut butter such as sunflower, sesame, soy, almond, cashew, hazelnut or peanut butter. Processes for the preparation of nut butters typically comprise wet or dry grinding roasted or unroasted nuts to a paste having a particle size suitable for the preparation of nut beverages.

In other embodiments, the plant matter may be a hydrolyzed suspension such as an oat milk or syrup. Processes for the preparation of such cereal suspensions typically comprise mixing an oat material (such as rolled oats, milled oats, oat flour or oatmeal) with water and treated enzymatically by amylases to hydrolyze starch followed by removal of suspended matter.

The vegetal base disclosed herein comprise water. Water is typically present in an amount balancing the amounts of other ingredients to 100% by weight. In an embodiment water is present in an amount between 40% and 90% by weight, for example, or from 60% to 90%, or from 40% to 45% or from 45% to 50% or from 50% to 55% or from 55% to 60% or from 60% to 65% or from 65% to 70% or from 70% to 75%, or from 75% to 80% or from 80% to 85% or from 80% to 85%.

The vegetal base disclosed herein optionally comprises at least one vegetal fat. Suitable examples of such fats are vegetal oils including, but not limited to, coconut oil, canola oil, soybean oil, sunflower oil, safflower oil, palm oil, palm kernel oil, olive oil, avocado oil and/or mixtures or combinations thereof. The vegetal oils may be selected from the group consisting of coconut oil, palm oil, palm kernel oil, and/or mixtures or combinations thereof. The vegetal oils may be a combination of coconut oil and sunflower oil. The vegetal fat may be coconut oil in higher concentrations, in embodiments the coconut oil may be provided in the form of or derived from coconut cream.

In one embodiment the vegetal fat is present in an amount of from 1% to 10% by weight, for example, from 2% to 8%, or from 2.5% to 9%, or from 2.9% to 8%, or from 3% to 7%, or from 2.5% to 3.5%. Compositions with such amounts of vegetal fat present good organoleptic properties.

The vegetal base preferably comprises at least one plant protein. Plant protein ingredients are known in the art and are commercially available. Plant protein ingredients can be, for example, a plant protein isolate, concentrate, or flour.

The term "protein concentrate", as used herein, generally refers to protein derived from plant source that has been extracted from the plant source and purified. Protein concentrate may comprise greater than or equal to about 40%, 50%, 60%, 70%, or 80%, or more total protein on a dry matter basis. The protein concentration of the protein concentrate may be increased by greater than or equal to about 20%, 30%, 40%, 50%, 60%, 70%, 80%, or more than the protein concentration of the plant. A protein concentrate may comprise a single type of protein or a combination of different types of proteins.

The term "protein isolate", as used herein, generally refers to protein derived from a plant source that has been extracted from the plant source and purified. A protein isolate may have a higher purity than a protein concentrate. A protein isolate may be formed by further processing a protein concentrate to increase the protein concentration. Protein isolate may comprise greater than or equal to about 80%, 90%, 95%, or more protein on a dry matter basis.

The plant protein in these compositions may be entirely from, or a portion of, a plant matter of the base. Non-limiting examples include oils, pastes, powders or flours of almonds, coconuts, pea, oat, fava or soy, all of which may contain plant proteins and may be added as components of the vegetal base, without being added, specifically, as a protein concentrate or isolate.

In one embodiment the plant protein is present in the vegetal base in an amount of from 1% to 10% by weight, for example, from 1% to 8%, or from 1% to 9%, or from 1% to 8%, or from 1.5% to 7%, or from 1.5% to 4%.

The vegetal base disclosed herein optionally may include at least one sweetening agent. Such sweetening agent may contribute to the consumer perceived sweetness properties of the composition. It may also provide a metabolic substrate for b) S. *thermophilus* strain CNCM I-2964.

The sweetening agent in the vegetal base may be entirely from, or a portion of, a plant matter of the base or may be in the form of added sugars.

In one embodiment said mixture further comprises plant and/or fruit juices. In one embodiment said base may be enriched or fortified with further plant based ingredients or other nutrients such as but not limited to vitamins, minerals, trace elements or other micronutrients.

### b) Milk base

The term "milk base" as used herein shall be taken to mean a liquid dairy milk composition. Preferably the composition comprises at least about 30 % (w/w) milk, more preferably at least about 50% (w/w) milk and even more preferably at least about 70% (w/w) milk. In embodiments, the composition comprises 30 % to 100% (w/w) milk. In embodiments, the composition comprises 50% to 100% (w/w) milk. In embodiments, the composition comprises 70% to 100% (w/w) milk. Preferably said milk is animal milk, goat, ewe, camel, mare or cow milk, and most preferably to cow milk. Preferably said milk(s) are heat-treated, typically pasteurized, to ensure sterility. Preferably said heat treatment is carried out prior to the preparation of the fermented dairy composition.

Preferably said milk comprises one or more of skimmed, partially-skimmed or non-skimmed milk. Preferably said milk or milks may be in liquid, powdered and/or concentrated form. In one embodiment said milk further comprises milk components preferably selected from the group consisting of cream, casein, caseinate (for example calcium or sodium caseinate), whey proteins notably in the form of a concentrate (WPC), milk proteins notably in the form of a concentrate (MPC), milk protein hydrolysates, and mixtures thereof. In one embodiment said mixture further comprises plant and/or fruit juices. In one embodiment said milk or milks may be enriched or fortified with further milk components or other nutrients such as but not limited to vitamins, minerals, trace elements or other micronutrients.

### c) Strain CNCM I-2964 content

In embodiments, the compositions of the invention comprise at least 10⁵ cfu/g, more preferably at least 10⁶ cfu/g, such as at least 10⁷ cfu/g, e.g. at least 10⁸ cfu/g, such as at least 10⁹ cfu/g, e.g. at least 10¹⁰ cfu/g, such as at least 10¹¹ cfu/g of strain CNCM I-2964 per gram of composition. In embodiments, the compositions of the invention comprise 10⁵ to 10¹², 10⁶ to 10¹¹ or 10⁶ to 10¹⁰ colony forming unit (CFU) of strain CNCM I-2964 per gram of composition.

### Fermented food product compositions

In embodiments, the total sugar content by weight dry matter of the fermented food product is from 0 to 10%.

In embodiments, the fermented food product comprises less than about 10 % w/w added sugar.

In one embodiment the added sugar is added to the milk and/or vegetal base in an amount of from 0.5% to 10% by weight, for example, from 0.5% to 8%, or from 0.5% to 7%, or from 0.5% to 6%, or from 0.5% to 5%, or from 0.5% to 4%, or from 0.5% to 3% or from 1% to 6%, or from 1% to 5%, or from 1% to 4%, or from 1% to 3%.

In one embodiment the added sugar is selected from the group consisting of sucrose, fructose, saccharose, glucose, maltodextrin, dextrose, sorbitol, corn syrup, or a mixture thereof. A corn syrup sweetening agent may be, for example, a high fructose corn syrup, a corn syrup solid, cane sugar, beetroot sugar, honey, agave, maple syrup, or a combination thereof. In embodiments, the fermented food product is free from, or do not comprise, added sugar.

In embodiments, the fermented food product comprises 0 to 1 % w/w texturizing agents selected from the group consisting of modified starch, gelatin, agar agar, and/or carrageenan.

In embodiments, the fermented food product is free from, or do not comprise, texturizing agents selected from the group consisting of modified starch, gelatin, agar agar, and/or carrageenan.

Preferably the fermented food product comprises above about 0.3 g per 100 g by weight free lactic acid, more preferably above about 0.7 g or 0.6 g per 100 g by weight free lactic acid. In embodiments, the composition comprises 0.3 g to 0.7 grams per 100 g by weight free lactic acid.

Preferably the fermented food product comprises a protein content, preferably at least about 2.5%, more preferably at least about 3% or 3.5% (w/w). Preferably the composition has a pH equal to or lower than 5, preferably between about 3 and about 4.5 and more preferably between about 3.5 and about 4.5.

Preferably the fermented food product has a viscosity lower than 200 mPa.s, more preferably lower than 100 mPa.s and most preferably lower that 60 mPa.s, at 10°C, at a shear rate of 64 s⁻¹. In embodiments, the composition has a viscosity range of 1 to 200 mPa.s, 1 to 100 mPa.s, or 1 to 60 mPa.s, at 10°C, at a shear rate of 64 s⁻¹. In embodiments, the composition has a viscosity range of 10 to 200 mPa.s, 10 to 100 mPa.s, or 10 to 60 mPa.s, at 10°C, at a shear rate of 64 s⁻¹. In embodiments, the composition has a viscosity range of 30 to 200 mPa.s, 30 to 100 mPa.s, or 30 to 60 mPa.s, at 10°C, at a shear rate of 64 s⁻¹.

The fermented food product according to embodiments of the invention is preferably a product selected from the group comprising yogurt, set yogurt, stirred yogurt, pourable yogurt, yogurt drink, frozen yogurt, kefir, buttermilk, quark, sour cream, fresh cheese, cheese and plant-based alternatives thereto. In one embodiment the composition according to embodiments of the invention is a drinkable composition, more preferably a fermented milk drink such as but not limited to a yogurt drink, kefir and plant-based alternatives thereto. In an alternative embodiment the composition according to embodiments of the invention is a composition that is spoonable, such as a set or stirred yogurt or plant-based alternatives thereto.

In one embodiment the fermented food product is a strained fermented food product. The strained fermented food product ion preferably has the following contents (% by weight):
- from 8.5% to 11.0% of protein
- from 0.0% to 8.0% of fat, for example from 0.0% to 3.5% or from 3.5% to 8.0%
- optionally from 0.00% to 4.20% of lactose, for example from 2.80% to 4.20%
The pH of the strained fermented food product can for example be of from 3.80 to 4.65.

### Intermediate Preparations

In embodiments, the food product further comprises an intermediate preparation. Intermediate preparations are known to the one skilled in the art. They are typically used to modify the taste, mouthfeel and/or texture of a food product, for example of a fermented food product. They can used also to introduce some additives such as nutrients. They typically comprise sweetening agents, flavors, color modifiers, cereals and/or fruit. Intermediate fruit preparations are for example slurries or fruit preparations. Flavors include for example fruit flavors, vanilla flavors, caramel flavors, coffee flavors, chocolate flavors.

Fruit preparations typically comprise fruits, as used herein the term "fruit" refers to any fruit form, including for example full fruits, pieces, purees, concentrates, juices etc.

The intermediate preparation or slurry typically comprises a stabilizing agent, having at least one stabilizer. The stabilizing agent can comprise at least two stabilizers. Such stabilizers are known to the one skilled in the art. They typically help in avoiding phase separation of solids, for examples of fruits or fruits extracts and/or in avoiding syneresis. They typically provide some viscosity to the composition, for example a viscosity (Bostwick viscosity at 20°C) of from 1 to 20 cm/min, preferably of from 4 to 12 cm/min.

The stabilizing system or the stabilizer can for example be a starch, a pectin, a guar, a xanthan, a carrageenan, a locust bean gum, or a mixture thereof. The amount of stabilizing system is typically of from 0.5 to 5% by weight.

The intermediate preparation can typically comprise organoleptic modifiers. Such ingredients are known by the one skilled in the art.

The organoleptic modifiers can be for example sweetening agents different from sugar, coloring agents, cereals and/or cereal extracts.

Examples of sweetening agents are ingredients referred to as High Intensity Sweeteners, such as sucralose, acesulfamK, aspartam, saccharine.

Examples of fruits include for example strawberry, peach, apricot, mango, apple, pear, raspberry, blueberry, blackberry, passion, cherry, and mixtures or associations thereof, such as peach-passion.

The fruits can be for example provided as:
- frozen fruit cubes, for example 10 mm fruit cubes, for example Individual Quick Frozen fruit cubes, for example strawberry, peach, apricot, mango, apple, pear fruit cubes or mixtures thereof,
- Aseptic fruit cubes, for example 10 mm fruit cubes, for example strawberry, peach, apricot, mango, apple or pear fruit cubes or mixtures thereof,
- fruit purees, for example fruit purees concentrated from 2 to 5 times, preferably 3 times, for example aseptic fruit purees, for example strawberry, peach, apricot, mango, raspberry, blueberry or apple fruit purees or mixtures thereof,
- single aseptic fruit purees, for example strawberry, raspberry, peach, apricot, blueberry or apple single aseptic fruit purees or mixture thereof,
- frozen whole fruits, for example Individual Quick Frozen whole fruits, for example blueberry, raspberry or blackberry frozen whole fruits, or mixtures thereof,
- mixtures thereof.

The ingredients and/or components of the intermediate preparation and the amounts thereof can be typically such that the composition has a brix degree of from 1 to 65 brix, for example from 1 to 10 brix, or from 10 to 15 brix, or from 15 to 20 brix, or from 20 to 25 brix, or from 25 to 30 brix, or from 30 to 35 brix, or from 35 to 40 brix, or from 40 to 45 brix, or from 45 to 50 brix, or from 50 to 55 brix, or from 55 to 60 brix, or from 55 to 60 brix, or from 60 to 65 brix. The Brix degree corresponds to the sugar content of a preparation. Methods for measuring the brix degree are well-known by the person skilled in the art. When measuring the brix degree of a fruit preparation, the fruit preparation is filtered on a sieve of 1 mm, and the supernatant (thus, without fruit pieces) is collected.

A fruit preparation can for example comprise fruit in an amount of from 30% to 80% by weight, for example from 50 to 70% by weight.

The intermediate preparation can comprise water. It is mentioned that a part of the water can come from ingredients used to prepare the fruit preparation, for example from fruits or fruit extracts or from a phosphoric acid solution.

The fruit preparation can comprise pH modification agents such as citric acid. The fruit preparation can have a pH of from 2.5 to 5, preferably of from 2.8 to 4.2.

Typically a fruit preparation can be added in an amount of 5-35% by weight with reference to the total amount of composition. In embodiments the composition of the invention comprises up to about 30% (w/w) of said intermediate preparation, e.g. up to about 10%, 15%, 20%, 25% (w/w). In one embodiment, the composition according to embodiments of the invention comprise 1% to 30% (w/w) of said intermediate preparation. In alternative embodiments, the composition according to embodiments of the invention comprise 1% to 25% (w/w) of said intermediate preparation. In further alternative embodiments, the composition according to embodiments of the invention comprise 1% to 20% (w/w) of said intermediate preparation. In additional embodiments, the composition according to embodiments of the invention comprise 1% to 15% (w/w) of said intermediate preparation. In further additional embodiments, the composition according to embodiments of the invention comprise 1% to 10% (w/w) of said intermediate preparation.

### Packaged Products

Preferably the composition, according to embodiments of the invention is provided in a sealed or sealable container containing about 50 g, 60 g, 70 g, 75 g, 80 g, 85 g, 90 g, 95 g, 100 g, 105 g, 110 g, 115 g, 120 g, 125 g, 130 g, 135 g, 140 g, 145 g, 150 g, 200 g, 300 g, 320 g or 500 g or about 1 oz, 2 oz, 3 oz, 4 oz, 5 oz, 6 oz or 12 oz product by weight.

In embodiments, the composition, according to embodiments of the invention is provided in a sealed or sealable container containing about 50 g to 500 g, 60 g to 500 g, 70 g to 500 g, 75 g to 500 g, 80 g to 500 g , 85 g to 500 g, 90 g to 500 g, 95 g to 500 g, 100 g to 500 g, 105 g to 500 g, 110 g to 500 g, 115 g to 500 g, 120 g to 500 g, 125 g to 500 g, 130 g to 500 g, 135 g to 500 g, 140 g to 500 g, 145 g to 500 g, 150 g to 500 g, 200 g to 500 g, 300 g to 500 g, 320 g to 500 g or 500 g product by weight. In embodiments, the composition, according to embodiments of the invention is provided in a sealed or sealable container containing about 1 oz to 12 oz, 2 oz to 12 oz, 3 oz to 12 oz, 4 oz to 12 oz, 5 oz to 12 oz, 6 oz to 12 oz or 12 oz product by weight.

Preferably the compositions, according to embodiments of the invention, may be stored, transported and/or distributed at a temperature of from 1°C to 10°C for at least about 30 days, at least about 60 days or at least about 90 days from packaging and remain suitable for consumption.

Preferably, the composition is a packaged product that comprises at least 10⁵, preferably 10⁶, more preferably at least 10⁷ and most preferably at least 10⁸ colony forming unit (CFU) of *S. thermophilus* strain CNCM I-2964 per gram (g) of composition according to embodiments of the invention subsequent to storage, transport and/or distribution at a temperature of from 1°C to 10°C for at least about 30 days, at least about 60 days or at least about 90 days from packaging.

In embodiments, the composition is a packaged product that comprises 10⁵ to 10¹² or 10⁶ to 10¹¹ or 10⁶ to 10¹⁰ colony forming unit (CFU) of S. *thermophilus* strain CNCM I-2964 per gram (g) of composition according to embodiments of the invention subsequent to storage, transport and/or distribution at a temperature of from 1°C to 10°C for at least about 30 days, at least about 60 days or at least about 90 days from packaging.

### Inoculum compositions

The bacterium as described herein is useful as starter culture in the preparation of food products, such as fermented food products. Accordingly, in a third aspect embodiment the present invention provides an inoculum comprising the *Streptococcus thermophilus* strain CNCM I-2964 that is suitable for the preparation of fermented food products. The inoculum of the invention is suitable for the direct inoculation of the strain CNCM I-2964 into a composition comprising a substrate to provide fermented food products of the invention, typically without the need for a culture step prior to the said direct inoculation.

Typically the inoculum further comprises excipient or carriers, the selection of which is within the scope of the skilled person but may include buffers or culture media. The inoculum may optionally comprise further components such as cryoprotectants, lyoprotectants, antioxidants, preservatives and/or additives including nutrients such as yeast extracts, cysteine, sugars and vitamins.

Typically the inoculum is for use in the preparation of fermented food product, according in one embodiment the inoculum of the invention may be provided to the food product in quantities of up to about 500 mg/l.

Typically the inoculum is fresh, frozen, dried, freeze-dried or lyophilized. The inoculum may be in liquid, dry, spray-dried or solid form. It is particularly preferred that the inoculum is in liquid form. The inoculum may be defrosted and/or dispersed in liquid (e.g. water) prior to inoculation into a substrate.

In embodiments, the inoculum comprises at least 10⁹ cfu, e.g. at least 10¹⁰ cfu, such as at least 10¹¹ cfu of strain CNCM I-2964 per gram of inoculum composition. In embodiments, the inoculum comprises 10⁹ to 10¹² colony forming unit (CFU), or more preferably 10¹⁰ to 10¹² colony forming unit (CFU) of strain CNCM I-2964 per gram of inoculum.

Preferably the inoculum comprising strain CNCM I-2964 is substantially pure.

In a further embodiment the present invention provides a mixture or kit of parts of the inoculum of the invention together with inoculum of *Bifidobacterium* and/or lactic acid bacteria.

Examples of *Bifidobacterium* that can be used include but are not limited to *Bifidobacterium animalis* (for example *Bifidobacterium animalis* subsp. *animalis* or *Bifidobacterium animalis* subsp. *lactis*); *Bifidobacterium longum*; *Bifidobacterium breve*; *Bifidobacterium bifidum.* Examples of lactic acid bacteria that can be used include but are not limited to *Lactobacilli* (for example *Lactobacillus acidophilus, Lactobacillus buchneri, Lactobacillus delbrueckii,* in particular *L. delbrueckii* subsp. *bulgaricus* or *lactis, Lactobacillus casei, Lactobacillus plantarum, Lactobacillus reuteri, Lactobacillus johnsonii, Lactobacillus helveticus, Lactobacillus brevis, Lactobacillus rhamnosus); Lactococci* (for example *Lactococcus lactis,* typically *Lactococcus lactis* subsp. *lactis* or *Lactococcus lactis* subsp. *cremoris*)*.* Preferably the inoculum mixture further comprises *Lactobacillus* and/or *Streptococcus.* For the preparation of yogurt, the inoculum mixture typically comprises *Lactobacillus bulgaricus* (also referred to as *Lactobacillus delbruckeii* subsp. *bulgaricus*) and *Streptococcus thermophilus,* optionally with additional microorganisms such as but not limited to probiotic species or other species that may provide desirable organoleptic or other qualities to the composition, e.g. *Lactococcus lactis.*

In one embodiment, the inoculum may be used for the preparation of a food product. This use may comprise a step of adding the inoculum to a milk and/or vegetal base.

### Methods for the preparation of fermented food products

The bacteria as provided herein are suitable for use in the preparation of fermented food products. Accordingly in a fourth aspect the present invention also relates to the intended use of the *Streptococcus thermophilus* strain CNCM I-2964 for the preparation of a food product.

In one embodiment, the use of the strain CNCM I-2964 for the preparation of a food product comprises a step of adding the strain CNCM I-2964 to a milk base and/or vegetal base.

In one embodiment the present invention provides a process for the preparation of a fermented food product comprising providing a mixture comprising the strain CNCM I-2964 and fermenting.

Accordingly in one embodiment the present invention provides a process comprising the following steps:
i) providing a mixture comprising:
   a) milk and/or vegetal base, and
   b) *Streptococcus thermophilus* strain CNCM I-2964,
ii) fermentation of said mixture to provide a fermented food product.

### i) Mixtures

Mixtures of the invention may be prepared by mixing or otherwise combining a) milk and/or vegetal base with b) strain CNCM I-2964. Preferably, the composition comprises at least about 30 % (w/w) milk and/or vegetal base, more preferably at least about 50% (w/w) milk and/or vegetal base and even more preferably at least about 70% (w/w) milk and/or vegetal base. In embodiments, the composition comprises at 30 % to 99% (w/w) milk and/or vegetal base. In other embodiments, the composition comprises 50% to 100% (w/w) milk and/or vegetal base. In other embodiments, the composition comprises 70% to 100% (w/w) milk and/or vegetal base.

### ia) Vegetal base

In one embodiment, the vegetal base is an aqueous suspension comprising water and plant-matter selected from the group consisting of legumes, nuts, seeds, cereals and/or combination thereof.

In embodiments, the plant-matter comprises legumes, and most preferably, pulse or pulses. In other embodiments, the pulses are selected from the group consisting of split peas, field peas, dry peas, lentil, chickpeas, garbanzo bean, konda, navy bean, white navy bean, white pea bean, pea bean, cow pea, horse bean, haricot, pinot bean, mottled bean, small red bean, red Mexican bean, kidney bean, black bean, black turtle bean, cranberry bean, roman bean, speckled sugar bean, lima bean, faba bean, Madagascar bean, green gram, mung bean, green bean, black gram, urad dal, soy and/or lupin. In preferred embodiments, the pulses are pea and/or chickpea.

In other embodiments, the nuts are selected from the group consisting of almonds, cashews, pecans, macadamias, hazelnuts, pistachio, walnuts and/or combinations thereof.

In other embodiments, the seeds are selected from the group consisting of hemp, pumpkin, quinoa, sesame, tiger nut, flax, chia, sunflower, coconut and/or combinations thereof.

In other embodiments, said cereals are selected from the group consisting of wheat, rye, spelt, barley, oat, millet, sorghum, rice, teff and/or combinations thereof.

Processes for the preparation of such suspensions are known in the art and typically comprise mechanical and/or enzymatic disruption of the plant-matter and hydration and/or combination with a solution, followed by mechanical separation of an aqueous fraction from starchy and/or fibrous matter, e.g., by decantering, centrifugation or filtration.

For example, the plant-matter may be milled, ground, soaked, dehulled, mixed with water, optionally enzymatic hydrolysed and/or homogenized etc. in order to produce a suitable aqueous composition.

In other embodiments, the plant matter may be a seed or nut butter such as sunflower, sesame, soy, almond, cashew, hazelnut or peanut butter. Processes for the preparation of nut butters typically comprise wet or dry grinding roasted or unroasted nuts to a paste having a particle size suitable for the preparation of nut beverages.

In other embodiments, the plant matter may be a hydrolyzed cereal suspension such as an oat milk or syrup. Processes for the preparation of such cereal suspensions typically comprise mixing an oat material (such as rolled oats, milled oats, oat flour or oatmeal) with water and treated enzymatically by amylases to hydrolyze starch followed by removal of suspended matter.

### ia) Milk base

Preferably, the milk base is animal milk, more preferably goat, ewe, camel, mare or cow milk, and most preferably to cow milk.

Preferably, the milk comprises one or more of skimmed, partially-skimmed or non-skimmed milk. Preferably, the milk or milks may be in liquid, powdered and/or concentrated form. In one embodiment, the milk further comprises milk components preferably selected from the group consisting of cream, casein, caseinate (for example calcium or sodium caseinate), whey proteins notably in the form of a concentrate (WPC), milk proteins in the form of a concentrate (MPC), milk protein hydrolysates, and mixtures thereof. In one embodiment, the milk or milks may be enriched or fortified with further milk components or other nutrients such as but not limited to vitamins, minerals, trace elements or other micronutrients.

### Milk and/or vegetal base preparation

In embodiments the milk and/or vegetal base may be adjusted to a pre-defined standard prior to the preparation of the composition. Preferably said standard is a defined nutritional standard of proteins, carbohydrates, fats and/or micronutrients. Standardization of substances may be carried out by the addition of milk and/or vegetal base components to reach said predefined standard.

Preferably fermented products are prepared using milk and/or vegetal base that has been subjected to heat treatment at least equivalent to pasteurization to ensure safety of consumption of the final product in regard to pathogens. Preferably said heat treatment is carried out prior to the preparation of the composition.

Methods for carrying out such heat treatments are known to the one skilled in the art and include, pasteurization, ultra-heat treatment and sterilization. The selection of appropriate time and temperature combinations is within the scope of the skilled person and is selected according to the final product properties and shelf-life requirements.

In one embodiment the base preparation is carried out by means of the following successive steps:
1) standardization of nutritional levels of the raw material so as to obtain a standardized substance,
2) an optional stage of enrichment with dried matter of the standardized substance obtained in the preceding stage, so as to obtain an enriched substance,
3) preheating of the substance, so as to obtain a starting substance,
4) pasteurization and holding of the starting substance obtained in the preceding stage, so as to obtain a pasteurized and held substance,
5) an optional stage of homogenization of the pasteurized and held substance obtained in the preceding stage, so as to obtain a pasteurized, held and optionally homogenized substance,
6) initial cooling of the pasteurized, held and optionally homogenized substance obtained in the preceding stage, so as to obtain a pasteurized starting substance that has been held, optionally homogenized, and cooled down.

As used herein "standardization of substances" is taken to mean a stage of bringing the quantity of a given substance present in the starting substance to a pre-determined level.

As used herein "holding" is taken to mean a rapid heating and maintenance of temperature of the base and makes it possible to destroy the vegetative microbial flora, including pathogenic forms. Its typical duration is from 4 to 10 minutes, in particular from 5 to 8 minutes, and in particular approximately 6 minutes.

As used herein "homogenization" is taken to mean the dispersion of the fatty substances in the liquid substance into small fat globules. The homogenization is carried out for example at a pressure of 100 to 280 bars, in particular 100 to 250 bars, in particular 100 to 200 bars, in particular approximately 200 bars. This homogenization stage is purely optional. It is in particular absent from the production process of products with 0% fatty substances.

### ib) Streptococcus thermophilus strain CNCM I-2964

The mixture further comprises strain CNCM I-2964.

According to a further embodiment of the process for the preparation of a fermented food product as defined above, the mixture comprising S. *thermophilus* strain CNCM I-2964 further comprises at least one, two, three or more strains of *Bifidobacterium* and/or lactic acid bacteria. The selection of suitable *Bifidobacterium* strains is within the scope of the skilled person and is typically a probiotic lactic acid bacteria. Examples of *Bifidobacterium* that can be used include but are not limited to *Bifidobacterium animalis* (for example *Bifidobacterium animalis* subsp. *animalis* or *Bifidobacterium animalis* subsp. *lactis*); *Bifidobacterium longum*; *Bifidobacterium breve; Bifidobacterium bifidum.*

The selection of suitable lactic acid bacteria strains is within the scope of the skilled person and is typically a thermophillic lactic acid bacteria. Examples of lactic acid bacteria that can be used include but are not limited to *Lactobacilli* (for example *Lactobacillus acidophilus, Lactobacillus buchneri, Lactobacillus delbruckeii,* in particular *L. delbrueckii* subsp. *bulgaricus* or *lactis, Lactobacillus casei, Lactobacillus plantarum, Lactobacillus reuteri, Lactobacillus johnsonii, Lactobacillus helveticus, Lactobacillus brevis, Lactobacillus rhamnosus); Lactococci* (for example *Lactococcus lactis,* typically *Lactococcus lactis* subsp. *lactis* or *Lactococcus lactis* subsp. *cremoris*)*.* Typically a mixture or association of a plurality of species of lactic acid bacteria may be used, typically a mixture or association of *Lactobacillus* and *Streptococcus.* For the preparation of yogurt this typically includes *Lactobacillus bulgaricus* and *Streptococcus thermophilus,* optionally with additional microorganisms such as but not limited to probiotic species or other species that may provide desirable organoleptic or other qualities to the composition, e.g. *Lactococcus lactis.*

Accordingly in one embodiment the mixture further comprises at least one strain of *Lactobacillus bulgaricus* and optionally one or more strains of *Lactococcus lactis* and/or *Bifidobacterium.*

### ii) Fermentation

Typically a fermented product is prepared by culture of a substrate at a suitable temperature with suitable microorganisms to provide a reduction in pH, preferably to a pH equal to or lower than 5, preferably between about 3 and 4.7; more preferably between about 3.5 and about 4.7. The pH can be adjusted by controlling the fermentation by the microorganism and stopping it when appropriate, for example by cooling.

Suitable temperatures for such fermentation are typically about 36°C to about 44°C and the temperature is maintained for an incubation time sufficient to provide the desired reduction in pH. For the preparation of a fermented food product the temperature at the start of fermentation is typically about 36°C to about 43°C, in particular about 37°C to about 40°C, the temperature at the end of fermentation is typically about 37°C to about 44°C, in particular about 38°C to about 41°C. The fermentation time is typically about 6 to about 11 hours.

Subsequent to the fermentation the fermented product is cooled. Optionally a stage of intermediate cooling of the fermented food product may be performed to provide a pre-cooled fermented food product having a temperature of between about 22°C and about 4°C. Typically the intermediate cooling time is about 1 hour to about 4 hours, in particular about 1 hour 30 minutes to about 2 hours. The pre-cooled fermented food product is typically stored for up to 40 hours or less.

Preferably a stage of final cooling of the fermented product is performed such that the temperature at the start of the final cooling is less than about 22°C and the temperature at the end of the final cooling is about 4°C to about 10°C. The cooled product may then be stored, transported and/or distributed at a temperature from about 1°C to about 10°C for at least about 30 days, at least about 60 days or at least about 90 days.

According to a further embodiment, the process for the preparation of a fermented food product as defined above optionally comprises a stage of stirring at a pressure of at least 20 bars, or performing a dynamic smoothing, to obtain a composition having the desired viscosity, typically a viscosity of up to 20 mPa.s. Stirring or dynamic smoothing operations provide some shear to composition that typically allow a viscosity drop. Such operations are known by the one skilled in the art, and can be operated with conventional appropriate equipment. This stage is typically performed at cold temperature, for example at a temperature of form 1 °C to 20°C. Without intending to be bound to any theory, it is believed that applying some shear at cold temperature, typically by stirring at high pressure or by performing a dynamic smoothing, can lead to a fluid gel formation within the composition, that provides improved stability even at a low viscosity of up to 20 mPa.s.

Alternatively, according to a further embodiment, the process for the preparation of a fermented food product as defined above optionally comprises a stage of acid whey or plant-based whey alternative removal to provide a "strained fermented food composition". In this step an acid whey composition is separated from the curd resulting from the protein coagulation due to acidification during fermentation. Thus one obtains:
- a fermented food product, typically comprising the proteins coagulum, referred to as a strained fermented food product, and
- an acid whey or plant-based whey alternative by-product

Such separation steps are known by the one skilled in art, for example in processes of making "greek yogurts". The separation can for example be carried out by reverse osmosis, ultrafiltration, or centrifugal separation. The separation step can be performed for example at a temperature of from 30°C to 45°C.

According to a further embodiment, the process for the preparation of a fermented food product as defined above optionally comprises a stage of addition of an intermediate preparation as described above prior or subsequent to fermentation, said intermediate preparation typically comprising a preparation of fruits and/or cereals and/or additives such as flavorings and/or colourings.

The invention will be further illustrated by the following non-limiting Figures and Example.

### Description of the figures

Figure 1. Acidification curve of test and control products.
Figure 2. Viscosity over shelf-life (5, 14, 30 days) at 10 and 90 seconds.
Figure 3. Acidification curves pea and soy yoghurts type products.

### Examples

### Example 1: Preparation of a fermented dairy milk product using S. thermophilus strain CNCM I-2964

Probiotic yoghurt type fermented dairy milk products were prepared by fermenting a dairy milk base of sweetened, homogenized and pasteurized whole milk (4.1% w/w proteins, 3.2% w/w fat, 6% w/w added sucrose).

A "Test Product 1" was prepared by fermentation with a "Test Culture" CNCM I-2964 and a probiotic yoghurt starter culture (2 strains of *L. bulgaricus,* 1 *S*. *thermophilus,* 1 *L. lactis,* and 1 *B. animalis* subsp. *Lactis).*

A "Control Product 1" was prepared by fermentation with the probiotic yoghurt starter culture only "Control Culture" (2 strains of *L. bulgaricus,* 1 *S*. *thermophilus,* 1 *L. lactis,* and 1 *B. animalis* subsp. *Lactis*) without CNCM I-2964.

The cultures were provided in frozen pellet form and defrosted prior to use at 38°C, the liquid cultures were then added to the milk mixture at 0.05% CNCM I-2964 v/v and 0.005% probiotic yoghurt starter culture v/v, respectively.

Fermentation was carried out at 37°C, monitored using a CINAC probe and stopped at pH 4.65 (8-10 hours) by rapid cooling and the product was stored at 10°C.

The viscosity was measured repeatedly until 30 days by applying a regular shearing strength using a rheometer with 2 co-axial cylinders. Mobile n°2 together with pot n°2 were used to perform the analysis. 64 s-1 shearing was applied during 90 seconds on the test product at 10°C. Values on viscosity at 10 and 90 seconds were used.

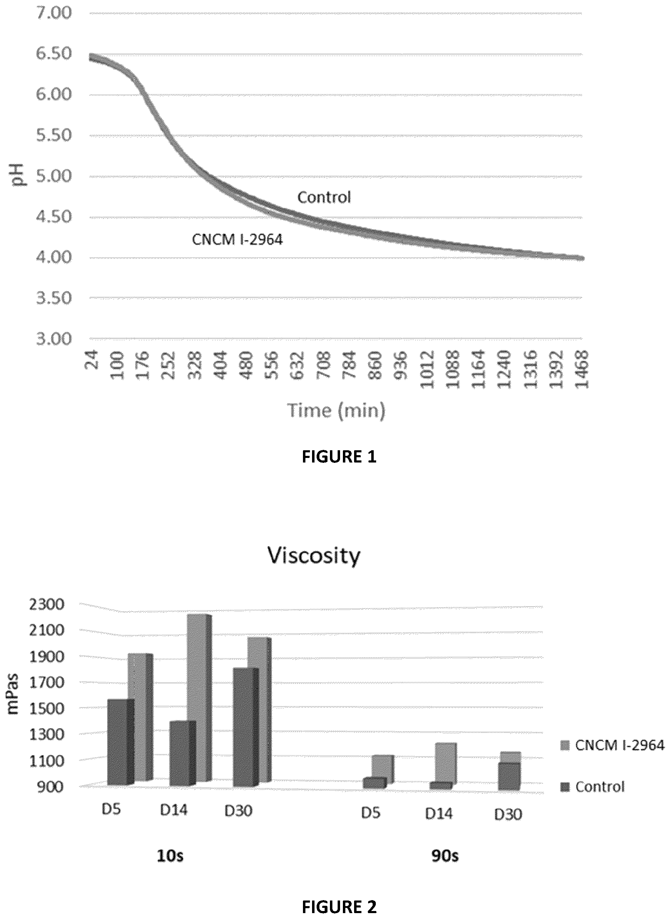

CNCM I-2964 demonstrated an improvement in texture (viscosity increase) without impacting fermentation time, as illustrated by Figures 1 & 2.

### Example 2: Use of strain CNCM I-2964 at different concentrations in fermented 0% fat milk with texturizers

In order to test the texturizing properties of CNCM I-2964, the "Test Culture" was used to ferment a fat free milk base, with limited texturizing agents to emulate a typical "clean label" type yoghurt product.

Low fat probiotic yoghurt type fermented dairy milk products were prepared by fermenting, storing and analyzing a dairy milk base of unsweetened homogenized and pasteurized skimmed milk (4.2% w/w proteins, 1% w/w native potato starch, 1.7% w/w inulin, 0.8% w/w acacia gum, 0% w/w fat) according to Example 1, pH 4.65 was typically reached in 7-8 hours.

A "Test Product 2" was prepared by fermentation with the "Test Culture" as in Example 1.

A "Test Product 3" was prepared by fermentation with the "Test Culture" as in Example 1 but with the dosage of the culture in the milk base reduced by half to 0.0025% vol/vol.

A "Control Product 2" was prepared by fermentation with the "Control Culture" as in Example 1. As provided in Table 1, a consistent improvement in texture was provided by the addition of CNCM I-2964 to the products, and this was maintained during 30 days of shelf life.

**Table 1. Viscosity over shelf-life (5, 14, 30 days).**

| | Viscosity 10s (m.Pa) | | | | Viscosity 90s (m.Pa) | | |
|---|---|---|---|---|---|---|---|
| Products | D5 | D14 | D30 | | D5 | D14 | D30 |
| Control (Control product 2) | 1121 | 1176 | 1145 | | 767 | 797 | 765 |
| CNCM I-2964 (0.005%) (Test product 2) | 1330 | 1236 | 1190 | | 1077 | 959 | 872 |
| CNCM I-2964 (0.0025%) (Test product 3) | 1406 | 1295 | 1195 | | 1025 | 923 | 852 |

These results show the excellent texturizing properties of CNCM I-2964 in yoghurt-type products.

### Example 3: Consumer test with and without strain CNCM I-2964 in sugar reduced formulas

In addition to the improvements in texture provided by CNCM I-2964,it was further investigated whether CNCM I-2964 could improve sweetness perception that could enable a consumer benefit in sugar reduction.

A blinded consumer test with 132 participants was conducted using sequential monadic sweetness liking evaluation of control and test products prepared according to Example 1.

A "clean label" dairy milk yogurt formula (milk, cane sugar, skimmed milk powder, cream, native tapioca starch, pectin and flavouring agent) was prepared according to Example 1 in vanilla and strawberry flavours. The latter, were prepared at various added sugar levels and reduced by 1% or 2% total sugar as compared to a commercial standard product of the same flavour.
Test product 4: Test Culture total sugars reduced by 2% w/w
Control product 4: Control Culture total sugars reduced by 2% w/w
Test product 5: Test Culture total sugars reduced by 1% w/w
Control product 5: Control Culture total sugars reduced by 1% w/w

Finally, the above test and control products were compared to a commercial standard product made with the Control Culture and dairy milk base comprising additional texturizing agents (modified potato starch, gelatin, milk protein concentrate, agar agar, acacia gum, carrageenan) and added sucrose in order to determine if they were equivalent to typical commercially available products (3.8 % w/w proteins, 1.5% w/w fat, 4.4% w/w added sucrose).

**Table 2. Consumer sweetness liking.**

| | Commercial standard product | Test product 5 (-1% total sugars) | Control product 5 (-1% total sugars) | Test product 4 (-2% total sugars) | Control product 4 (-2% total sugars |
|---|---|---|---|---|---|
| Vanilla | 7.0 | 7.3 | 7.1 | 7.3 | 6.8 |
| Strawberry | 7.4 | 7.2 | 7.0 | 7.4 | 6.7 |

Surprisingly, it was observed that the addition of the strain CNCM I-2964 consistently improved the consumer sweetness liking of the test products over the control products that did not contain CNCM I-2964.

Furthermore, in vanilla flavoured products, the test products consistently performed better than even the higher sugar commercial standard.

Accordingly, the addition of the strain CNCM I-2964 to fermented milk products enables a reduction in sugar content while providing a good sweetness experience for the consumer.

### Example 4: Preparation of fermented plant-based yoghurt alternative products using strain CNCM I-2964

The suitability of the strain CNCM I-2964 for the preparation of plant-based yogurt alternatives was determined by fermenting plant bases according to Example 1.

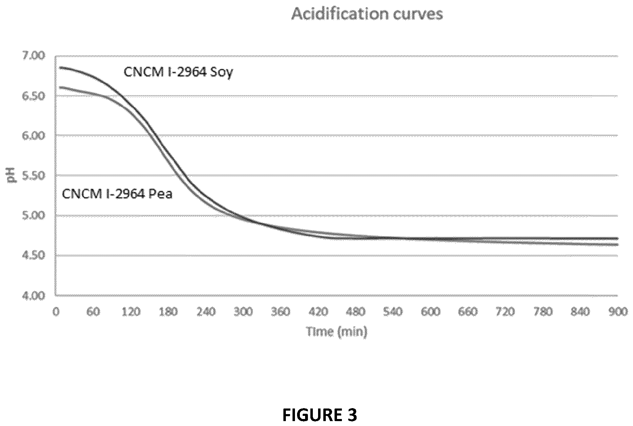

A pea base (11.90% w/w pea protein isolate, 0.85% w/w table sugar added, 2.0% w/w rapeseed oil, 6% w/w oat syrup, 79.25 % w/w water) and alternatively a commercially available soy milk (without added sugar) were both successfully fermented as demonstrated in Figure 3.

### Example 5: Use of strain CNCM I-2964 in fermented 0% fat milk

In order to test the texturizing properties of CNCM I-2964, the "Test Culture" was used to ferment a fat free milk base.

Low fat probiotic yoghurt type fermented dairy milk products (without the addition of texturizing agents) were prepared by fermenting, storing and analyzing a dairy milk base of unsweetened homogenized and pasteurized skimmed milk (5,15% w/w proteins, 0% w/w fat) according to Example 1, pH 4.65 was typically reached in 7-8 hours.

A "Test Product 6" was created by fermenting with the "Test Culture" as in Example 1.

Similarly, a "Control Product 6" was made using the "Control Culture" from Example 1.

Viscosity was measured as provided above.

As shown in Table 3, adding CNCM I-2964 consistently improved the texture of the products and this improvement persisted for 15 days.

**Table 3. Viscosity over shelf-life (5, 14 days).**

| | Viscosity 10s (m.Pa) | | | Viscosity 90s (m.Pa) | |
|---|---|---|---|---|---|
| Products | D5 | D14 | | D5 | D14 |
| Control (Control product 6) | 1809 | 1679 | | 1032 | 1097 |
| CNCM I-2964 (0.005%) (Test product 6) | 1960 | 2261 | | 1202 | 1420 |

These findings demonstrate the outstanding texturizing capabilities of CNCM I-2964 in yoghurt-like products.

## Claims

1. A *Streptococcus thermophilus* strain deposited at the CNCM under reference number I-2964 .

2. A composition comprising S. *thermophilus* strain CNCM I-2964.

3. The composition according to claim 2 wherein said composition is a food product.

4. The composition according to claim 3 wherein said product is a fermented food product.

5. The composition according to claim 4 comprising a) fermented milk and/or vegetal base and b) S. *thermophilus* strain CNCM I-2964.

6. The composition according to claim 2 wherein said composition is an inoculum.

7. The composition according to claim 6 wherein said composition is fresh, frozen, dried, freeze-dried or lyophilized.

8. The composition according to any one of preceding claims comprising at least 10⁵ CFU/g of *S*. *thermophilus* strain CNCM I-2964.

9. The composition according to any one of preceding claims further comprising at least one, two, three or more strains of *Bifidobacterium* or lactic acid bacteria.

10. The composition according to any one of preceding claims comprising 0-10% w/w sugar.

11. A method for the preparation of a fermented food product comprising:
i) providing a mixture comprising:
a) milk and/or vegetal base, and
b) *S*. *thermophilus* strain CNCM I-2964,
ii) fermentation of said mixture to provide a fermented food product.
